# EUROPÄISCHE PATENTANMELDUNG

(11) **EP 4 559 378 A1**
(43) Veröffentlichungstag der Anmeldung: **28.05.2025**
(21) Anmeldenummer: 23020523.9
(22) Anmeldetag: 27.11.2023
(51) Int. Cl.: A61B 5/00, G01R 33/563

(54) **VORRICHTUNG ZUR ERZEUGUNG VON SCHWINGUNGEN IN MENSCHLICHEM GEWEBE FÜR DIE MAGNETRESONANZ-ELASTOGRAPHIE**

(71) Anmelder: Medizinische Universität Wien, 1090 Wien (AT)
(72) Erfinder: Kiss, Lorenz, A-1190 Wien (AT); Schmid, Albrecht Ingo, A-1170 Wien (AT)
(74) Vertreter: SONN Patentanwälte GmbH & Co KG

(57) **Zusammenfassung**

Vorrichtung zur Erzeugung von Schwingungen in menschlichem Gewebe für die Magnetresonanz-Elastographie, umfassend ein Gehäuse mit einem mittleren Gehäuseabschnitt (2) und zwei äußeren Gehäuseabschnitten (3,4), eine Turbine (6), die im mittleren Gehäuseabschnitt (2) um eine Drehachsen (13) drehbar gelagert ist und durch ein in den mittleren Gehäuseabschnitt (2) einströmendes Fluid antreibbar ist, und zwei von der Turbine (6) angetriebene Unwuchtelemente (7,8), die in den äußeren Gehäuseabschnitten (3,4) jeweils um eine zur Drehachse (13) der Turbine (6) fluchtende Drehachse (13) drehbar gelagert sind, sodass die Turbine (6) in axialer Richtung zwischen den beiden Unwuchtelementen (7,8) angeordnet ist.

## Beschreibung

Die Erfindung betrifft eine Vorrichtung zur Erzeugung von Schwingungen in menschlichem Gewebe für die Magnetresonanz-Elastographie.

Schwingungserzeuger, auch als Transducer oder Wellengeber bezeichnet, werden in der Magnetresonanz-Elastographie verwendet, um mechanische Wellen in das zu untersuchende Gewebe zu leiten, wobei die Scherwellen aufgrund der niedrigeren Phasengeschwindigkeit relevant sind. Diese führen beim Auftreffen zu einer Verschiebungen im Mikrometerbereich, welche mit Hilfe des Magnetresonanztomographen sichtbar gemacht werden können. Bipolare Gradientenpulse erlauben die Kodierung der Wellenamplitude in der Phase des MR-Signals. Wiederholt man die Bildaufnahme zeitlich versetzt zur Schwingung ergeben sich Wellenmuster, die als Grundlage zur Berechnung der Elastizität des Gewebes dienen. Die mechanischen Eigenschaften, wie z.B. die Elastizität, des Gewebes und deren Änderung sind ein wichtiges Indiz zur Diagnose oder Einstufung diverser Krankheiten, wie z.B. Fibrose. Weiters sind Steifigkeitsänderungen der Muskulatur eine direkte Funktion der Muskelspannung und somit ein Maß für den Aktivitätszustand.

Bei der Nutzung des Schwingungserzeugers für die Magnetresonanz-Elastographie sind mehrere Schlüsselfaktoren zu berücksichtigen.

Es ist entscheidend, dass der Schwingungserzeuger bei einer stabilen Frequenz arbeitet, die je nach spezifischer Anwendung üblicherweise zwischen 20 und 200 Hz variiert. Niederfrequente Wellen werden weniger stark gedämpft und können daher tiefer in den Körper eindringen, während höhere Frequenzen durch kürzere Wellenlängen eine präzisere Lokalisierung und genauere Darstellung der Gewebesteifigkeit ermöglichen. Es ist zu beachten, dass der Schermodul direkt mit der Quadratwurzel der Wellenausbreitungsgeschwindigkeit zusammenhängt, welche jedoch nicht für alle Frequenzen konstant ist. Daher kommt auch die Multifrequenzelastographie zum Einsatz.

Die Frequenzstabilität während der gesamten Messung ist von großer Bedeutung. Ebenso kritisch ist die erzeugte Wellenamplitude, um eine adäquate Eindringtiefe ins Gewebe zu gewährleisten. Abhängig von der Tiefe und den Eigenschaften des untersuchten Gewebes sowie von vorhandenen Strukturen und Grenzschichten muss eine geeignete Kombination von Amplitude und Frequenz gefunden werden. Höhere Frequenzen erleiden geringere Abschwächungen, bieten aber eine kürzere Eindringtiefe. Daher ist es vorteilhaft, wenn der Schwingungserzeuger eine breite Auswahl an Frequenz- und Amplitudeneinstellungen bietet. Weitere essenzielle Merkmale umfassen die MR-Kompatibilität, also die Abwesenheit von magnetischen oder metallischen Bestandteilen, Biokompatibilität, um sicherzustellen, dass das Material des Schwingungserzeugers keinen negativen Einfluss auf lebendes Gewebe hat, sowie eine angemessene Größe. Der Schwingungserzeuger sollte so dimensioniert sein, dass er zwischen der RF-Spule und dem Patienten Platz findet, um die Distanz zwischen diesen so gering wie möglich zu halten.

Aus der EP 3384844 A1 ist ein mechanischer Aktuator für die Magnetresonanz-Elastographie bekannt geworden, der das Prinzip der Zentrifugalkraft zur Schwingungserzeugung nutzt. Der mechanische Aktuator umfasst einen passiven Antrieb mit einem rotierenden Turbinenvibrator, der mit einem exzentrischen Gewicht ausgestattet ist, wobei der Turbinenvibrator durch ein Fluid, wie z.B. komprimierte Luft oder Wasser, angetrieben wird, und einen aktiven Antrieb, der so konfiguriert ist, dass er den Druck des Fluids, das den Turbinenvibrator antreibt, steuert. Nachteilig hierbei ist die koaxialen Anordnung von Turbine und exzentrischem Gewicht derart, dass die Turbine das exzentrische Gewicht umgibt. Dies führt zu einer hohen Bauweise, sodass der Aktuator nicht ohne weiteres zwischen der RF-Spule und dem Patienten angeordnet werden kann.

Bei mechanisch erregten Schwingungserzeugern, die mit einem exzentrischen Gewicht arbeiten, besteht ein Zielkonflikt zwischen der Notwendigkeit einer kompakten, flachen Bauweise und der Bereitstellung einer ausreichenden Masse zur Erzeugung wirksamer Zentrifugalkräfte. Ein flacher Schwingungserzeuger bietet weniger Raum für die notwendige Masse, um ausreichende Schwingungsamplituden zu erzeugen. Eine größere Bauform wiederum vergrößert den Abstand zwischen der RF-Spule und dem Patienten, was zu einer Abschwächung des empfangenen Signals und einer Beeinträchtigung der Bildqualität führt.

Daher ist es ein Ziel der vorliegenden Erfindung, einen Schwingungserzeuger für die Magnetresonanz-Elastographie zu verbessern, indem der oben genannte Zielkonflikt aufgelöst wird. Der Schwingungserzeuger soll eine flache Bauweise mit ausreichender Masse für die Schwingungserzeugung vereinen, um effektive Zentrifugalkräfte zu erzeugen, ohne die Anordnung zwischen RF-Spule und Patient zu beeinträchtigen.

Zur Lösung dieser Aufgabe sieht die Erfindung gemäß einem ersten Aspekt einen Schwingungserzeuger vor, umfassend ein Gehäuse mit einem mittleren Gehäuseabschnitt und zwei äußeren Gehäuseabschnitten, eine Turbine, die im mittleren Gehäuseabschnitt um eine Drehachse drehbar gelagert ist und durch ein in den mittleren Gehäuseabschnitt einströmendes Fluid antreibbar ist, und zwei von der Turbine angetriebene Unwuchtelemente, die in den äußeren Gehäuseabschnitten jeweils um eine zur Drehachse der Turbine fluchtende Drehachse drehbar gelagert sind, sodass die Turbine in axialer Richtung zwischen den beiden Unwuchtelementen angeordnet ist.

Durch die axiale Anordnung der Turbine zwischen den Unwuchtelementen ergibt sich eine deutlich geringere Bauhöhe des Schwingungserzeugers. Gleichzeitig wird durch die Verwendung von zwei Unwuchtelementen im Gegensatz zu einem einzelnen exzentrischen Gewicht eine ausreichende Schwingungsmasse bereitgestellt.

Die Ausbildung des Schwingungserzeugers mit der Turbine in der Mitte und der symmetrischen Anordnung der Unwuchtelementen auf beiden Seiten der Turbine ermöglicht zudem eine ausgewogene Verteilung von Masse und Schwingungskräften.

Dadurch, dass die Turbine in einem mittleren Gehäuseabschnitt und daher getrennt von den in den äußeren Gehäuseabschnitten angeordneten Unwuchtelementen angeordnet ist, wird die Fluidzu- und -abfuhr zur bzw. von der Turbine nicht durch die Unwuchtelemente beeinträchtigt. Die fluidbetriebene Turbine bietet einen zuverlässigen und einstellbaren Mechanismus zur Vibrationserzeugung, der eine präzise Steuerung der Frequenz und Amplitude der Vibrationen ermöglicht, was für eine hochwertige Bildgebung entscheidend ist.

Die Übertragung der Drehbewegung von der Turbinen auf die beiden Unwuchtelemente erfolgt gemäß einer bevorzugten Weiterbildung der Erfindung dadurch, dass die Unwuchtelemente jeweils mittels einer formschlüssigen Steckverbindung drehfest mit der Turbine gekoppelt sind.

Die Steckverbindung erlaubt ein einfaches Aufstecken und Abziehen des jeweiligen Unwuchtelements auf die bzw. von der Turbine, wobei im aufgesteckten bzw. aufgeschobenen Zustand ein in Drehrichtung wirksamer Formschluss hergestellt ist. Durch die Steckverbindung wird einerseits eine einfache Montage gewährleistet und andererseits ein Austausch der Unwuchtelemente ermöglicht. Letzteres kann beispielsweise von Nutzen sein, um je nach Bedarf Unwuchtelemente mit unterschiedlicher exzentrischer Masse einzusetzen. Außerdem wird dadurch ein Wechsel der Turbine erleichtert, um auf diese Weise bei Bedarf unterschiedlichste Turbinentypen verwendet werden können.

Bevorzugt ist hierbei vorgesehen, dass die formschlüssige Steckverbindung an einem Element ausgewählt aus der Turbine und dem Unwuchtelement einen Achsenabschnitt mit nicht kreisförmigem Querschnitt und an dem anderen Element ausgewählt aus der Turbine und dem Unwuchtelement eine Aufnahme für das formschlüssige axiale Einstecken des Achsenabschnitts aufweist. Der nicht kreisförmige Querschnitt kann beispielsweise ein dreieckiger, rechteckiger, quadratischer oder sternförmiger Querschnitt sein. Eine besonders vorteilhafte Ausbildung mit sternförmigem Querschnitt umfasst einen Querschnitt mit der Form eines Innen- oder Außensechsrunds ("Torx^{®}"). Eine derartige Formgebung des Querschnitts gewährleistet eine gute Kraftübertragung bei geringem Materialverschleiß.

Eine weitere bevorzuge Ausbildung sieht vor, dass der nicht kreisförmige Querschnitt eine Rotationssymmetrie der Ordnung ≥ 2 aufweist. Dies erlaubt es, die Unwuchtelemente in verschiedenen Winkelpositionen formschlüssig mit der Turbine zu verbinden, wodurch beispielsweise die Möglichkeit eines Winkelversatzes zwischen den zwei Unwuchtelementen geschaffen wird, um bei Bedarf Schwingungsüberlagerungen zu erzeugen.

Die Unwuchtelemente können vorteilhafterweise axiale Bohrungen zur Aufnahme von Gewichtsstäben aufweisen. Die Bohrungen sind hierbei exzentrisch angeordnet, sodass durch Variation des Gewichts der Gewichtsstäbe oder durch Variation der Anzahl an Gewichtsstäben die Gesamtmasse der Unwucht der Unwuchtelemente eingestellt werden kann. Die Gewichtsstäbe können beispielsweise aus einem Kunststoff mit gegenüber dem Material der Unwuchtelemente höherer Dichte, wie z.B. PTFE, bestehen. Vorzugsweise weisen die Unwuchtelemente mehrere, in Umfangsrichtung verteilte Bohrungen für die Aufnahme von Gewichtsstäben auf. Durch Variieren unterschiedlicher Kombinationen aus Gewichten kann neben der Wahl der Frequenz die Kraftentwicklung des Schwingungserzeugers gesteuert werden.

Wie bereits erwähnt, ist das Gehäuse in einen mittleren Gehäuseabschnitt und zwei äußere Gehäuseabschnitte unterteilt. Um hierbei eine Trennung des mittleren Gehäuseabschnitts von den äußeren Gehäuseabschnitten zu erreichen, sieht eine bevorzugte Weiterbildung der Erfindung vor, dass der mittlere Gehäuseabschnitt eine gegenüber den Unwuchtelementen abgeschlossene, insbesondere fluiddichte, Kammer zur Aufnahme der Turbine begrenzt. Dies stellt sicher, dass das für den Antrieb der Turbine in den mittleren Gehäuseabschnitt bzw. in die von diesem begrenzte Kammer eingeleitete Fluid nicht mit den Unwuchtelementen in Berührung kommt. Die hierfür erforderliche Abdichtung kann beispielweise eine Dichtschnur umfassen, die an den einander zugewandten Dichtflächen von zwei Gehäusehälften des Gehäuses angeordnet sind.

Der Antrieb der Turbine kann grundsätzlich mit beliebigen Fluiden erfolgen. Vorzugsweise wird Wasser oder Luft verwendet. Die Kammer zur Aufnahme der Turbine ist daher wasser- oder luftdicht ausgeführt.

Um die von den Unwuchtelementen erzeugten Vibrationen möglichst unmittelbar auf die zu untersuchende Körperregion des Patienten zur Wirkung bringen zu können, ist bevorzugt vorgesehen, dass die Unwuchtelemente an ihren gegenüberliegenden Endbereichen in Wälzlagern gelagert sind, welche die durch die Rotation der Unwuchtelemente erzeugten Schwingungen auf das Gehäuse übertragen. Die Wälzlager sind zu diesem Zweck zum Beispiel in geeigneten ringförmigen Aufnahmenuten des Gehäuses unbeweglich, wie z.B. formschlüssig, aufgenommen.

Bevorzugt bilden die der Turbine jeweils zugewandten Wälzlager eine Achsendurchführung zwischen der die Turbine aufnehmenden Kammer des mittleren Gehäuseabschnitts und den die Unwuchtelemente aufnehmenden Kammern der äußeren Gehäuseabschnitte aus.

Um die MR-Kompatibilität des Schwingungserzeugers zu gewährleisen, ist bevorzugt vorgesehen, dass die Vorrichtung einschließlich des Gehäuses, der Turbine, der Unwuchtelemente und der Wälzlager aus nicht-magnetisierbaren, insbesondere nicht-metallischen Materialien, wie z.B. Polymere (Polyamid, Polyurethan, Polypropylen, Polycarbonat), Keramik, nicht ferromagnetischen Metallen, besteht.

Gemäß einem weiteren Aspekt betrifft die vorliegende Erfindung die Verwendung einer Vorrichtung gemäß dem ersten Aspekt der Erfindung zur Erzeugung von Schwingungen in menschlichem Gewebe im Rahmen der Magnetresonanz-Elastographie. Der Schwingungserzeuger wird hierbei auf die zu untersuchende Körperregion des Patienten aufgelegt und in Betrieb gesetzt, sodass die erzeugten Vibrationen in die Körperregion eingebracht werden. Der Schwingungserzeuger wird insbesondere zwischen einer Erregerspule eines Magnetresonanztomographen und der zu untersuchenden Körperregion angeordnet.

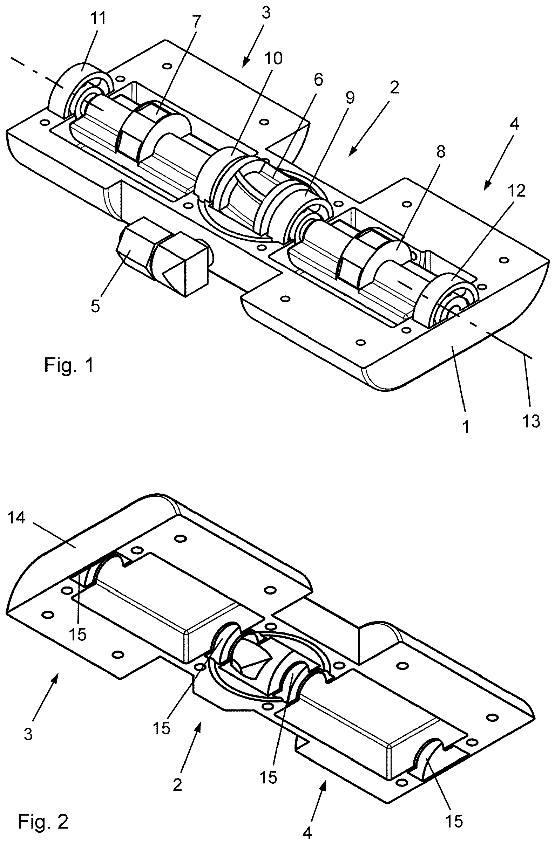

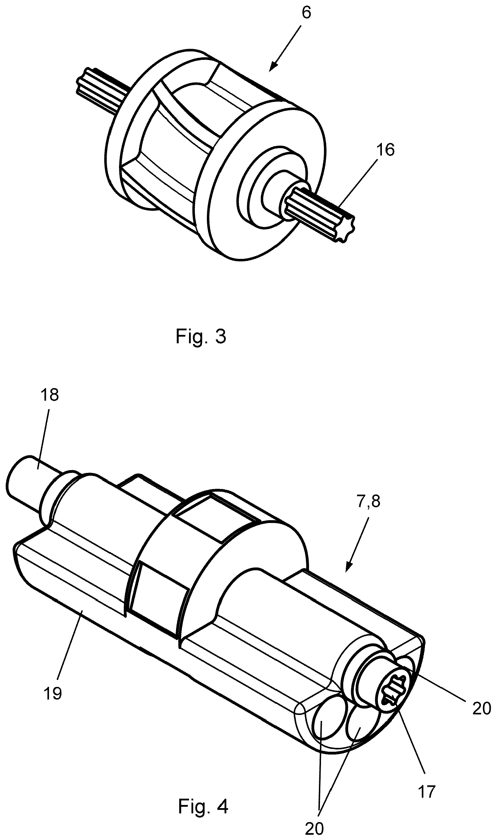

Die Erfindung wird nachfolgend anhand eines in der Zeichnung schematisch dargestellten Ausführungsbeispiels näher erläutert. In dieser zeigt Fig. 1 einen erfindungsgemäßen Schwingungserzeuger mit abgenommener oberer Gehäuseschale, Fig. 2 eine Darstellung der oberen Gehäuseschale, Fig. 3 die Turbine des in Fig. 1 dargestellten Schwingungserzeugers und Fig. 4 ein Unwuchtelement des in Fig. 1 dargestellten Schwingungserzeugers.

In Fig. 1 ist ein Schwingungserzeuger gemäß der Erfindung dargestellt, dessen Gehäuse aus zwei Gehäuseschalen besteht. Die untere Gehäuseschale ist in Fig. 1 mit 1 bezeichnet, wohingegen die obere Gehäuseschale abgenommen und nicht dargestellt ist, um die im Inneren des Gehäuses angeordneten Bauteile besser zeigen zu können. Das Gehäuse umfasst einen mittleren Gehäuseabschnitt 2 und zwei äußere Gehäuseabschnitte 3 und 4. Im mittleren Gehäuseabschnitt 2 ist eine Turbine 6 angeordnet, die um eine Achse 13 drehbar gelagert ist. Für den Antrieb der Turbine 6 ist ein Fluideinlass 5 vorgesehen, über den ein Antriebsfluid, wie z.B. Wasser oder Druckluft, in die vom mittleren Gehäuseabschnitt umschlossene Kammer einzuleiten, wo das Antriebsfluid auf die Turbinenschaufeln trifft und die Turbine zu Drehbewegungen antreibt. Das Antriebsfluid wird über einen in Fig. 1 nicht sichtbaren Fluidauslass aus der Kammer ausgeleitet.

Die Turbine treibt die beiden Unwuchtelemente 7 und 8 an, die in den beiden äußeren Gehäuseabschnitten 3 und 4 jeweils um dieselbe Drehachse 13 gelagert sind. Die Turbine 6 ist in axialer Richtung somit zwischen den beiden Unwuchtelementen 7 und 8 angeordnet. Wie anhand der Fig. 3 und 4 noch näher erläutert werden wird, sind die Turbine 6 und die Unwuchtelemente 7 und 8 mittels einer formschlüssigen Steckverbindung miteinander drehfest verbunden und bilden somit eine gemeinsame Drehachse aus. Die Lagerung erfolgt mittels der Wälzlager 9, 10, 11 und 12, die alle formschlüssig in ringförmigen Aufnahmenuten 15 der Gehäuseschalen 1 und 14 (Fig. 2) aufgenommen sind, sodass die von den Unwuchtelementen 7 und 8 erzeugten Vibrationen unmittelbar auf das Gehäuse übertragen werden.

Die Turbine 6 ist in Fig. 3 gesondert dargestellt und umfasst einen Achsenabschnitt 16 mit nicht kreisförmigem, nämlich sternförmigem, Querschnitt. Der Achsenabschnitt 16 wird in eine am Unwuchtelement 7 bzw. 8 angeordnete Aufnahme 17 (Fig. 4) eingesteckt, die einen dem Querschnitt des Achsenabschnitts 16 entsprechenden Querschnitt aufweist, sodass der Achsenabschnitt 16 formschlüssig in die Aufnahme 17 passt und eine drehfeste Verbindung hergestellt wird. Die Aufnahme 17 ist an einem Achsabschnitt 18 des Unwuchtelements 7 bzw. 8 ausgebildet.

Wie in Fig. 4 ersichtlich ist, umfasst das Unwuchtelement 7 bzw. 8 eine exzentrisches Masse 19, in der radial verteilt Bohrungen 20 vorgesehen sind, die der Aufnahme von Gewichtsstäben dienen.

## Patentansprüche

1. Vorrichtung zur Erzeugung von Schwingungen in menschlichem Gewebe für die Magnetresonanz-Elastographie, umfassend ein Gehäuse mit einem mittleren Gehäuseabschnitt (2) und zwei äußeren Gehäuseabschnitten (3,4), eine Turbine (6), die im mittleren Gehäuseabschnitt (2) um eine Drehachsen (13) drehbar gelagert ist und durch ein in den mittleren Gehäuseabschnitt (2) einströmendes Fluid antreibbar ist, und zwei von der Turbine (6) angetriebene Unwuchtelemente (7,8), die in den äußeren Gehäuseabschnitten (3,4) jeweils um eine zur Drehachse (13) der Turbine (6) fluchtende Drehachse (13) drehbar gelagert sind, sodass die Turbine (6) in axialer Richtung zwischen den beiden Unwuchtelementen (7,8) angeordnet ist.

2. Vorrichtung nach Anspruch 1, **dadurch gekennzeichnet, dass** die Unwuchtelemente (7,8) jeweils mittels einer formschlüssigen Steckverbindung drehfest mit der Turbine (6) gekoppelt sind.

3. Vorrichtung nach Anspruch 2, **dadurch gekennzeichnet, dass** die formschlüssige Steckverbindung an einem Element ausgewählt aus der Turbine (6) und dem Unwuchtelement (7,8) einen Achsenabschnitt (16) mit nicht kreisförmigem Querschnitt und an dem anderen Element ausgewählt aus der Turbine (6) und dem Unwuchtelement (7,8) eine Aufnahme (17) für das formschlüssige axiale Einstecken des Achsenabschnitts (!6) aufweist.

4. Vorrichtung nach Anspruch 3, **dadurch gekennzeichnet, dass** der nicht kreisförmige Querschnitt eine Rotationssymmetrie der Ordnung ≥ 2 aufweist.

5. Vorrichtung nach einem der Ansprüche 1 bis 4, **dadurch gekennzeichnet, dass** die Unwuchtelemente (7,8) axiale Bohrungen (20) zur Aufnahme von Gewichtsstäben aufweisen.

6. Vorrichtung nach einem der Ansprüche 1 bis 5, **dadurch gekennzeichnet, dass** der mittlere Gehäuseabschnitt (2) eine gegenüber den Unwuchtelementen (7,8) abgeschlossene, insbesondere fluiddichte, Kammer zur Aufnahme der Turbine (6) begrenzt.

7. Vorrichtung nach einem der Ansprüche 1 bis 6, **dadurch gekennzeichnet, dass** die Unwuchtelemente (7,8) an ihren gegenüberliegenden Endbereichen in Wälzlagern (9,10,11,12) gelagert sind, welche die durch die Rotation der Unwuchtelemente (7,8) erzeugten Schwingungen auf das Gehäuse übertragen.

8. Vorrichtung nach Anspruch 7, **dadurch gekennzeichnet, dass** die der Turbine (6) jeweils zugewandten Wälzlager (9, 10) eine Achsendurchführung zwischen der die Turbine (6) aufnehmenden Kammer des mittleren Gehäuseabschnitts (2) und den die Unwuchtelemente (7,8) aufnehmenden Kammern der äußeren Gehäuseabschnitte (3,4) ausbilden.

9. Vorrichtung nach einem der Ansprüche 1 bis 8, **dadurch gekennzeichnet, dass** die Vorrichtung einschließlich des Gehäuses (1,14), der Turbine (6), der Unwuchtelemente (7,8) und der Wälzlager (9,10,11,12) aus nicht-magnetisierbaren, insbesondere nicht-metallischen Materialien, wie z.B. Polymere (Polyamid, Polyurethan, Polypropylen, Polycarbonat), Keramik, nicht ferromagnetischen Metallen, besteht.

10. Verwendung einer Vorrichtung nach einem der Ansprüche 1 bis 9, zur Erzeugung von Schwingungen in menschlichem Gewebe im Rahmen der Magnetresonanz-Elastographie.
